# FASCICULE DE BREVET EUROPEEN

(11) **EP 1 234 573 B1**
(45) Date de publication et mention de la délivrance du brevet: **27.07.2005**
(21) Numéro de dépôt: 01400458.4
(22) Date de dépôt: 22.02.2001
(51) Int. Cl.: A61K 9/06, A61K 31/19, A61K 47/08, A61K 47/14, A61K 47/32, A61K 31/5415, A61K 31/542

(54) **Compositions pharmaceutiques à action anti-inflammatoire comprenant du kétoprofène**
Entzündungshemmende Arzneizubereitungen enthaltend Ketoprofen
Anti-inflammatory pharmaceutical compositions comprising ketoprofen

(43) Date de publication de la demande: 28.08.2002
(73) Titulaire: Menarini France S.A., 94550 Chevilly Larue (FR)
(72) Inventeur: Bertrand, Jean-Christophe, 92290 Chatenay Malabry (FR); Dubois, Martine, 75116 Paris (FR)
(74) Mandataire: Burtin, Jean-François

(56) Documents cités:
- WO-A-92/05768
- WO-A-92/05769
- DATABASE WPI Section Ch, Week 198539 Derwent Publications Ltd., London, GB; Class B05, AN 1985-239345 XP002148566 & JP 60 155111 A (HISAMITSU PHARM CO LTD), 15 août 1985 (1985-08-15)
- PATENT ABSTRACTS OF JAPAN vol. 1996, no. 04, 30 avril 1996 (1996-04-30) & JP 07 324027 A (KANEBO LTD), 12 décembre 1995 (1995-12-12)
- DATABASE WPI Section Ch, Week 199736 Derwent Publications Ltd., London, GB; Class B05, AN 1997-389349 XP002148567 & JP 09 169658 A (POLA CHEM IND INC), 30 juin 1997 (1997-06-30)

## Description

La présente invention concerne le domaine de la chimie thérapeutique et plus particulièrement celui des médicaments.

Elle a plus précisément pour objet de nouvelles compositions pharmaceutiques à action anti-inflammatoire sans effet secondaire gênant.

Elle a spécifiquement pour objet des compositions pharmaceutiques topiques renfermant à titre de principe actif un agent anti-inflammatoire du type non stéroïdien (AINS) et un agent photo protecteur en association ou en mélange avec un excipient ou un véhicule approprié pour l'usage externe.

Le problème que l'invention vise à résoudre est celui des effets secondaires liés à l'utilisation topique de médicaments anti-inflammatoire dont certains sont sensibles à la lumière, notamment à l'exposition à la lumière solaire et surtout à l'irradiation par les rayons UV.

L'exposition à la lumière solaire a pour effet d'entraîner la dégradation chimique des molécules et d'amener la formation de produits de décomposition dont on sait que certains sont impliqués dans des phénomènes de sensibilisation.

En outre, la décomposition de certains médicaments anti-inflammatoires par la lumière a pour effet de faire baisser le titre en principe actif dans des proportions importantes. C'est ainsi que l'irradiation de solutions d'AINS sous une lampe à UV émettant à une longueur d'onde de 254nm, de 312nm ou de 365nm conduit à des dégradations rapides et importantes. Les solutions perdent leur limpidité et présentent des colorations.

En outre, la dégradation par photolyse conduit à la formation de sous-produits qui possèdent un potentiel allergisant.

Ces problèmes ont déjà été évoqués pour l'acide 6,11-dihydro 11-oxo benzo [b,e] oxepinyl acétique (Tagawa H et Kubo S,Chem. Pharm.Bull 32 (1984) 3047) et surtout pour l'acide tiaprofénique (Bosca F. et al. J.Pharm sci. 81 (1992) p 181-182). Ces problèmes existent en particulier pour des préparations topiques à base de Kétoprofène. On a constaté en particulier que la molécule de Kétoprofène est instable lorsqu'elle est exposée à la lumière du jour. Dans ces conditions on détecte la formation d'au moins quatre produits de dégradation principaux: la 3-acetyl benzophénone et trois autres produits. La 3-acetyl benzophénone n'est pas dépourvue de toxicité et manifeste des propriétés irritantes.

Il était donc souhaitable de réaliser des formes pharmaceutiques destinées à l'usage topique, qui ne développent pas de phénomènes de sensibilisation ou de phototoxicité lorsqu'elles sont appliquées sur la peau ou les muqueuses de sujets susceptibles de s'exposer largement à la lumière solaire.

Une première approche a été réalisée avec des préparations topiques contenant des agents anti-inflammatoires et une qualité particulière de dioxyde de titane. Cette solution a été appliquée au cas du suprofen (brevet japonais JP97.169658 au nom de Pola Chemical Industries). Il en résulte des préparations blanchâtres, peu plaisantes.

JP60155111 et WO92/05769 divulguent des compositions pharmaceutiques à usage topique comprenant du kétoprofène associé à un filtre U.V. et/ou à un antioxydant.

Un objet que l'invention vise à résoudre est celui de réaliser des formes topiques et notamment des gels translucides contenant un agent anti-inflammatoire du type non stéroïdien, qui soient stables à la lumière ou à l'irradiation grâce à la présence d'un filtre anti-UV, qui soient dépourvues de potentiel irritatif, sensibilisant et/ou allergisant. Il est souhaitable aussi que les compositions selon l'invention possèdent sensiblement la même biodisponibilité que les compositions topiques ne contenant pas de filtres anti-UV.

En effet, il pourrait se faire qu'un filtre anti-UV tout en protégeant le principe actif contre la photolyse, modifie sensiblement la biodisponibilité et surtout la pénétration dermique de l'agent anti-inflammatoire et par-là affecte notablement son niveau d'activité.

Tous ces problèmes sont importants et il était nécessaire d'y apporter une solution.

L'invention consiste donc en des compositions pharmaceutiques selon les revendications 1-15, destinées à l'application sur la peau et les muqueuses, renfermant à titre de principe actif au moins un agent anti-inflammatoire du type non stéroïdien, associé à un composé filtrant les rayons UltraViolet et notamment les rayons UV-B, dans un excipient ou un véhicule inerte, non toxique, pharmaceutiquement acceptable, ledit composé filtrant les rayons UV et ledit excipient ou véhicule étant choisi de manière à ne pas affecter d'une manière négative les caractéristiques pharmacocinétiques du principe actif dans la préparation.

L'agent anti-inflammatoire est le Kétoprofène ou un de ses sels avec une base minérale ou organique.

Le composé filtrant les rayons ultraviolets est choisi parmi le dérivé éthoxylé de l'acide p-aminobenzoïque dénommé PEG 25 - PABA, commercialisé sous la dénomination UVINUL P25 (BASF) ou encore le produit dénommé sulisobenzone (UVINUL MS 40 de la BASF). Les filtres anti-UV sont ajoutés aux compositions en quantités allant de 0,5 à 10 % et de préférence de 1 à 5%.

Comme autres substances solubles dans l'eau on pourra citer en outre :
- les dérivés de benzophénone porteurs d'un groupe acide sulfurique par exemple l'acide 2-hydroxy 4-méthoxybenzophénone 5-sulfornique et ses sels (UVINUL MS 40)
- les dérivés de 3-benzylidène camphre porteurs d'un groupe sulfonique comme l'acide 4-(2-hoxo 3-bornylidèneméthyl) benzène sulfonique et l'acide 2-méthyl 5-(2-oxo 3-bornylidène) sulfonique et leurs sels.

Le catalogue CFTA donne les définitions suivantes pour ces produits :

| | Dénomination CFTA | Numéros CAS |
|---|---|---|
| Uvinul M 40 | Benzophenone-3 | 131-57-7 |
| Uvinul MS 40 | Benzophenone-4 | 4065-45-6 |
| Uvinul D 50 | Benzophenone-2 | 131-55-5 |
| Uvinul D 49 | Benzophenone-6 | 131-54-4 |
| Uvinul DS 49 | Benzophenone-9 | 3121-60-6 |
| Uvinul 400 | Benzophenone-1 | 131-56-6 |
| Uvinul M 493 | Benzophenone-11 | 1341-54-4 |
| Uvinul N 539 | Octocrylène | 6197-30-4 |
| Uvinul P 25 | PEG-25-PABA | 116242-27-4 |
| Uvinul T 150 | Octyl Triazone | 88122-99-0 |

**Tableau 1**

| Propriétés | Soluble dans | | Effet de filtre UV | | Emploi | |
|---|---|---|---|---|---|---|
| | eau | huiles | UV-A | UV-B | Protection solaire | Protection du produit |
| Uvinul M 40 | | X | X | X | X | X |
| Uvinul MS 40 | X | | X | X | X | X |
| Uvinul D 50 | | X | XX | X | X | X |
| Uvinul D 49 | | X | XX | X | X | X |
| Uvinul DS 49 | X | | XX | X | | X |
| Uvinul 400 | | X | X | XX | | X |
| Uvinul M493 | | X | XX | X | | X |
| Uvinul N 539 | | miscible | | X | X | X |
| Uvinul P 25 | X | | | X | X | X |
| Uvinul T 150 | | X | | XX | X | X |

**Emploi :** Les filtres UV sont utilisés dans de nombreux produits cosmétiques. Ils servent à la protection de la peau, à la protection des produits, en particulier de la couleur, du parfum ou du principe actif ainsi qu'à la protection des cheveux contre l'effet néfaste du rayonnement U.V.

Certains produits sont des absorbeurs d'UV-B typiques c'est-à-dire qu'ils présentent un maximum d'absorption dans la zone allant de 280 à 320 mm. La plupart des produits UVINUL en particulier les dérivés de benzophénone sont des filtres à large spectre.

Les filtres anti-UV sont de préférence des agents filtrant les rayons UV B car les rayons UV-B sont les plus énergétiques et ne sont pas arrêtés par l'atmosphère. Les filtres organiques lipophiles et hydrophiles sont utilisés de préférence car ils permettent d'obtenir des compositions transparentes et notamment des gels transparents.

Les essais ont montré qu'il existait une corrélation entre la concentration en filtre UV et la photo protection, dans la mesure où la concentration en filtre UV est au moins égale à 0,5 % et de préférence comprise entre 0,5 % et 5 %.

La concentration en agent anti-inflammatoire non stéroïdien varie de 1 à 10 % avec une concentration préférée comprise entre 2 et 5 %.

La protection porte notamment sur la résistance à la dégradation du Kétoprofène en fonction de la durée de l'irradiation et de la teneur en agent filtrant. Les lots témoin ne comportant pas d'agent filtrant, conservent une teneur résiduelle en Kétoprofène de 75 % après une irradiation d'une heure et de 42 % après une irradiation de trois heures. L'addition de 1 % d'un agent filtrant comme UVINUL ® P25 ramène ces valeurs respectivement à 95 et à 70 %.

On peut évaluer également la protection contre la photolyse par l'évolution de la teneur en 3-acétyl benzophénone en fonction de la durée d'irradiation. Une solution témoin de Kétoprofène donne naissance après une heure d'irradiation, à 8 % de 3-acétyl benzophénone et après trois heures d'irradiation, à 15 % de 3-acétyl benzophénone alors que la même solution additionnée de 1 % d'UVINUL ® P25 produit respectivement 2 % et 5 % de 3-acétyl benzophénone.

En outre, on a constaté que l'adjonction d'un filtre UV comme par exemple le produit dénommé UVINUL ® P25 est facile à réaliser, s'incorpore bien dans une préparation topique et contribue à augmenter la solubilité du Kétoprofène. Il en résulte dans le cas de gels une plus grande stabilité et en particulier une moindre tendance à la cristallisation du principe actif.

Dans les gels aqueux selon l'invention, l'agent gélifiant sera de préférence un polymère d'acide acrylique et notamment ceux définis sous le nom de Carbomères. A cet égard, on citera plus particulièrement les qualités pharmaceutiques de Carbomère comme celles commercialisées sous les marques Carbopol P940, 941, 980 (Goodrich).

D'autres agents gélifiants peuvent également être utilisés seuls ou incorporés dans des gels de Carbomères. C'est le cas en particulier des dérivés de la cellulose comme la méthylcellulose, l'éthylcellulose, l'hydroxyéthylcellulose, l'hydroxypropylcellulose, l'hydroxypropylméthylcellulose, la carboxyméthylcellulose, la carboxyméthylcéllulose réticulée et les produits similaires, des gommes végétales comme la gomme guar ou la gomme arabique, la gomme xanthane, les carrhagenates, les alginates ou l'acide hyaluronique.

Les compositions selon l'invention peuvent également inclure des agents antioxydants qui renforcent l'effet des filtres UV. On pourra citer à cet égard le butylhydroxy anisole (BHA) ou le terbutylparacresol, l'ascorbate de palmityle ou les tocophérols.

La valeur du pH des gels sera choisie de manière à obtenir la viscosité optimale. D'une manière générale le pH des compositions s'échelonne de 5 à 7 et de préférence de 5,5 à 6,5 où les Carbomères présentent une viscosité maximale tout en manifestant la meilleure tolérance cutanée. Le pH du gel est ajusté à ces valeurs par addition d'un composé basique minéral ou organique, comme la soude, l'ammoniaque, la mono-, la di- ou la triéthanolamine.

Les compositions selon l'invention pourront également contenir un ou plusieurs alcools aliphatiques qui contribuent à maintenir en solution le ou les principes actifs, évitent les problèmes de recristallisation et maintiennent la limpidité et la transparence de la préparation. Parmi ceux-ci on utilise de préférence l'éthanol à différentes concentrations, l'isopropanol, le butanol et/ou le terbutanol.

Les compositions pharmaceutiques selon l'invention peuvent en outre renfermer un parfum ou un agent odoriférant dont la qualité doit être de ne pas être allergisant. Les essences végétales comme celles de géranium rosat, de lavande, de lavandin, d'Ylang-Ylang ou de citronnelle répondent à ce critère.

Compositions pharmaceutiques contenant un agent anti-inflammatoire et un agent protecteur contre les UV.

### Exemple 1 Gel

| | |
|---|---|
| Kétoprofène | 2,50 Kg |
| Ethanol | 59,00 l soit 46,30 Kg |
| Huile essentielle de lavandin | présence |
| Carbomère P940 commercialisé sous la dénomination Synthalin K^{(R)} | 1,65 Kg |
| UVINUL P25^{(R)} | 4,00 Kg |
| Butylhydroxyanisole (BHA) | 0,050 Kg |
| Triethanolamine | qsp pH 5,3 |
| Eau purifiée qsp | 100,00 Kg |

### Exemple 2 Gel

| | |
|---|---|
| Kétoprofène | 2,50 Kg |
| Isopropanol | 59,00 l soit 46,30 Kg |
| Huile essentielle de lavandin | présence |
| Carbomère P940 | 1,75 Kg |
| UVINUL P25^{(R)} | 4,00 Kg |
| Butylhydroxyanisole (BHA) | 0,050 Kg |
| Triethanolamine | qsp pH 5,3 |
| Eau purifiée qsp | 100,00 Kg |

### Exemple 3 Gel

| | |
|---|---|
| Kétoprofène | 2,50 Kg |
| Ethanol | 40,00 l |
| Huile essentielle de lavandin | présence |
| Carbomère P940 | 1,50 Kg |
| UVINUL P25^{(R)} | 4,00 Kg |
| Butylhydroxyanisole | 0,05 Kg |
| Triethanolamine | qsp pH 5,3 |
| Eau purifiée qsp | 100,00 Kg |

### Exemple 4 Gel

| | |
|---|---|
| Kétoprofène | 2,50 Kg |
| Isopropanol | 40,00 l |
| Huile essentielle de lavandin | présence |
| Carbomère P940 | 1,50 Kg |
| UVINUL P25^{(R)} | 4,00 Kg |
| Butylhydroxyanisole | 0,05 Kg |
| Triethanolamine | qsp pH 5,3 |
| Eau purifiée qsp | 100,00 Kg |

### Exemple 5 Gel

| | |
|---|---|
| Kétoprofène | 2,50 Kg |
| Ethanol | 50,00 I soit 40,70 Kg |
| Huile essentielle de lavandin | présence |
| Carbomère P940 | 1,60 Kg |
| UVINUL P25^{(R)} | 4,00 Kg |
| Butylhydroxyanisole | 0,05 Kg |
| Triethanolamine | qsp pH 5,3 |
| Eau purifiée qsp | 100,00 Kg |

### Exemple 6 Gel

| | |
|---|---|
| Kétoprofène | 2,50 Kg |
| Ethanol | 40,00 l soit 32,50 Kg |
| Huile essentielle de lavandin | présence |
| Carbomère P940 | 1,60 Kg |
| UVINUL P25^{(R)} | 4,00 Kg |
| Butylhydroxyanisole | 0,05 Kg |
| Triethanolamine | qsp pH 5,3 |
| Eau purifiée qsp | 100,00 Kg |

### Exemple 7 Gel

| | |
|---|---|
| Kétoprofène | 2,50 Kg |
| Ethanol | 40,00 l soit 32,50 Kg |
| Huile essentielle de lavandin | présence |
| Carbomère P940 | 1,80 Kg |
| UVINUL P25^{(R)} | 4,00 Kg |
| Butylhydroxyanisole | 0,05 Kg |
| Triethanolamine | qsp pH 6,0 |
| Eau purifiée qsp | 100,00 Kg |

### Exemple 8 Gel

| | |
|---|---|
| Kétoprofène | 2,50 Kg |
| Ethanol | 40,00 l soit 32,50 Kg |
| Huile essentielle de lavandin | présence |
| Carbomère P940 | 1,50 Kg |
| UVINUL P25^{(R)} | 2,00 Kg |
| Butylhydroxyanisole | 0,05 Kg |
| Triethanolamine | qsp pH 5,3 |
| Eau purifiée qsp | 100,00 Kg |

### Exemple 9 Gel

| | |
|---|---|
| Kétoprofène | 2,50 Kg |
| Ethanol | 40,00 l soit 32,50 Kg |
| Huile essentielle de lavandin | présence |
| Carbomère P940 | 1,50 Kg |
| UVINUL P25^{(R)} | 4,00 Kg |
| Butylhydroxyanisole | 0,05 Kg |
| Triethanolamine | qsp pH 5,3 |
| Eau purifiée qsp | 100,00 Kg |

### Exemple 10 Gel de Tenoxicam

| Composition pour 100g | |
|---|---|
| Tenoxicam | 2,5 g |
| Carbomère 940 | 2 g |
| Ethanol à 96° | 40 ml |
| Huile essentielle de lavandin | 0,1 g |
| Butylhydroxyanisole | 0,05 g |
| p-Methoxycinnamate d'octyle* | 3 g |
| Transcutol P (Ph.Eur.3^{ème} ed.) | 15 g |
| Cremophor RH40 (USPNF) | 10 g |
| Trométamol q.s. | |
| Eau distillée q.s. | 100 g |

| | |
|---|---|
| *Eusolex 2292 Merck AG | |

### Exemple 11 Gel de Diclofenac

| Composition pour 100g | |
|---|---|
| Diclofenac | 2,5 g |
| Carbomère 940 | 1,8 g |
| Ethanol à 96° | 40 ml |
| Huile essentielle de lavandin | 0,1 g |
| Butylhydroxyanisole | 0,05 g |
| Sulisobenzone* | 4 g |
| Triethanolamine q.s. to pH6 | |
| Eau distillée q.s.p | 100 g |

| | |
|---|---|
| *UVINUL MS40 (BASF AG) ou Uvasorb S5 3V (SIGMA) | |

### Exemple 12

### a) - Etude de la photoprotection assurée aux solutions de Kétoprofène par un agent protecteur anti UV-B.

La solution de principe actif sans carbomère mais contenant un agent photoprotecteur ou sans agent photoprotecteur (solution témoin) a été soumise à une irradiation par une lampe émettrice de rayons UV-B à 312 nm.
Deux agents photoprotecteurs ont été testés :
⇒ UVINUL P25 - ester polyethoxyéthylique de l'acide 4-bis (polyethoxy) aminobenzoïque
⇒ EUSOLEX 232 - acide 2-phénylbenzimidazole 5-sulfonique.

On procède ensuite à une chromatographie en couche mince pour détecter la présence de produits de dégradation en fonction du temps et en fonction de la qualité.

On a procédé ensuite à un essai sur un gel contenant du Kétoprofène, et ayant la même couleur et la même viscosité qu'un produit du commerce.

Les résultats obtenus ont été les suivants :

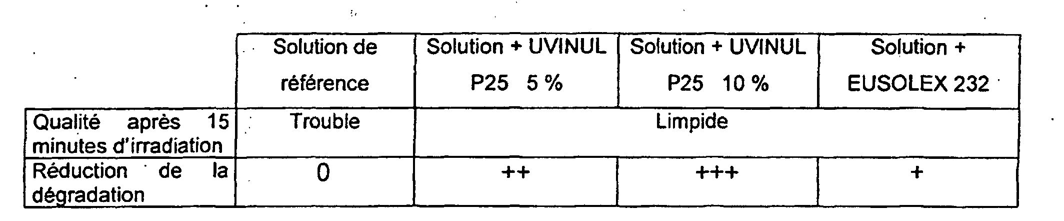

### b - Etude de la stabilité des gels contenant de l'UVINUL P25

Six formulations de gel ont été préparées, 3 gels contiennent un agent photoprotecteur, et trois contiennent un agent photoprotecteur associé à un agent antioxydant (BHA). Les gels sont conservés à 30° C à 60 % d'humidité relative ou à 40° C à 75 % d'humidité relative.
On teste la couleur du gel avant et après conservation. On détermine la pureté du Kétoprofène après conservation compte tenu des valeurs initiales.

Les résultats suivants ont été obtenus :
Etude de la stabilité à court terme du gel au Kétoprofène + UVINUL P

Test pour 6 formulations (3 lots avec un agent anti-UV avec ou sans BHA)

| CONDITIONS : 30° C 60 % R.H. - 40° 75 % R.H. | | | | |
|---|---|---|---|---|
| TEST | Initial | 1 mois | 2 mois | 3 mois |
| Couleur du gel Tous les lots/Condition | J 6 | J 4 | J 3 | J 3 |
| Essai Tous les lots/Condition | Environ 2,5 g /100 g | Environ 2,5 g/100 g | Environ 2,5 g/100 g | Environ 2,5 g/100 g |
| Pureté Tous les lots/Condition | Environ 100 % | Environ 100 % | Environ 100 % | Environ 100 % |
| Photo Efficacité Tous les lots/Condition | Inefficace | Comme initialement | Comme initialement | Comme initialement |
| J = mesure selon Eur. Ph. III (1997) | | | | |
| NOTE - La couleur du gel devient foncée d'une manière homogène sur tous les lots (négatifs), - L'essai et la pureté du gel de Kétoprofène sont stables (positifs) | | | | |

### c - Etude de la photolyse du Kétoprofène en l'absence ou en présence d'aqent filtrant pour les UV.

Un test d'irradiation a été effectué avec un gel de Kétoprofène soumis à une irradiation lumineuse de 175 KLux/h et de 75 W/h/M² (lampe au Xenon décrite dans les lignes de conduite (guidelines) de ICH comme susceptible de simuler la lumière du jour) qui représente une dose apte à simuler une exposition moyenne d'une journée en Europe.
On détermine l'évolution de la formation de produits de photolyse en fonction du temps. On a détecté l'apparition de quatre produits résultant de la photolyse.
L'addition de différents agents protecteurs permet de déterminer l'augmentation de la concentration de produits de photolyse en fonction de la concentration en agent photoprotecteur. Il apparaît clairement que par rapport aux gels témoin à base de Kétoprofène on peut supprimer dans une large proportion l'apparition de produits de photolyse qui sont irritants ou allergisants.

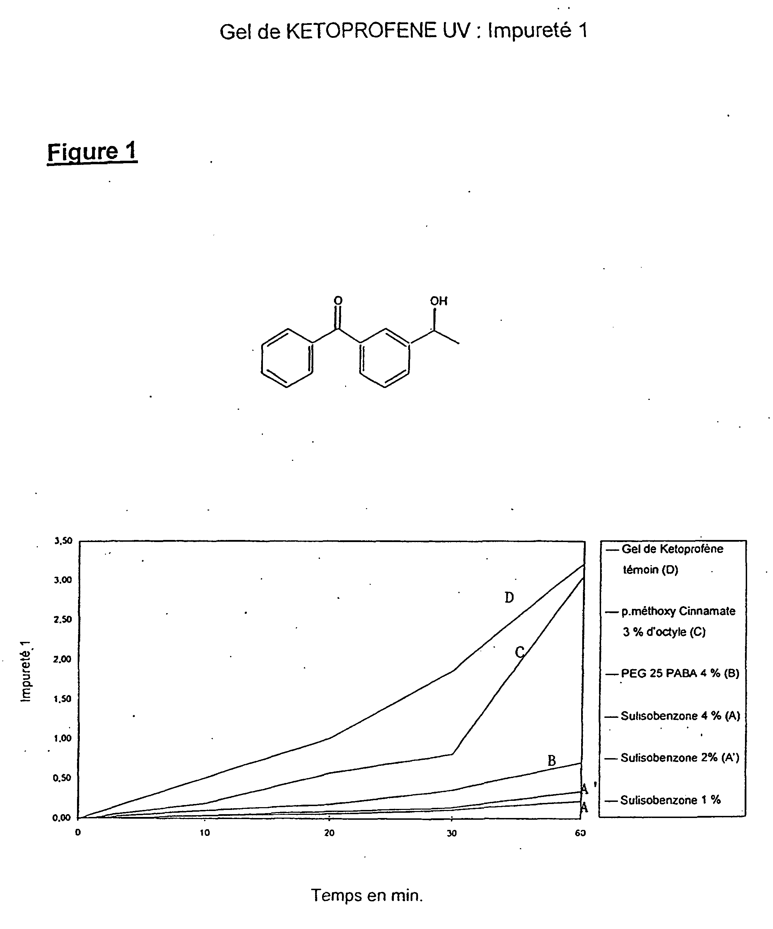

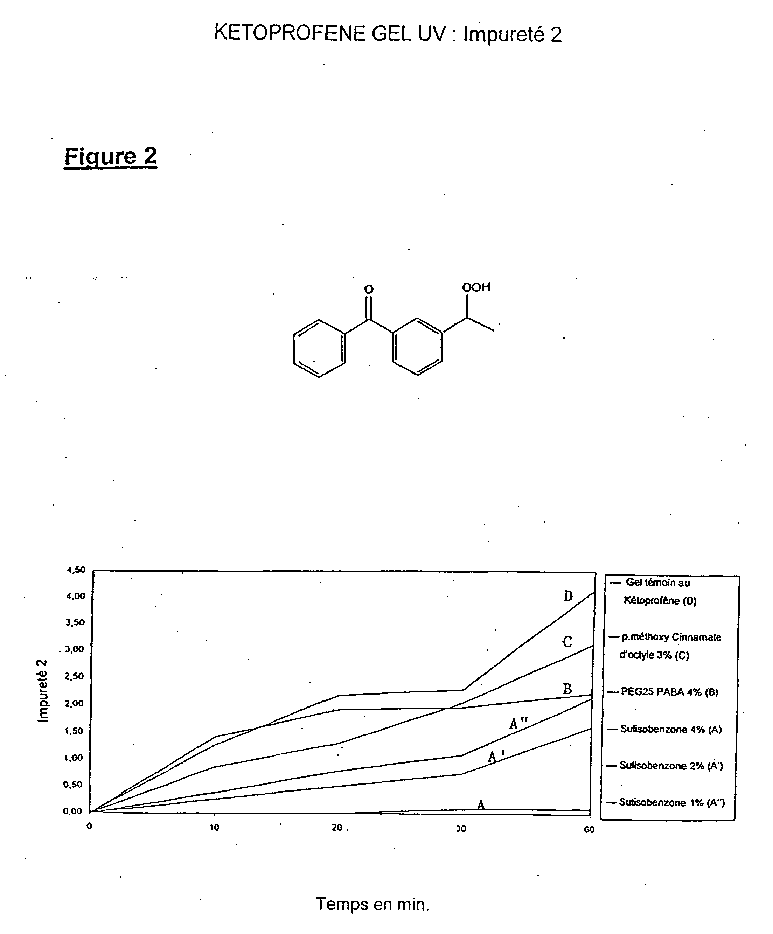

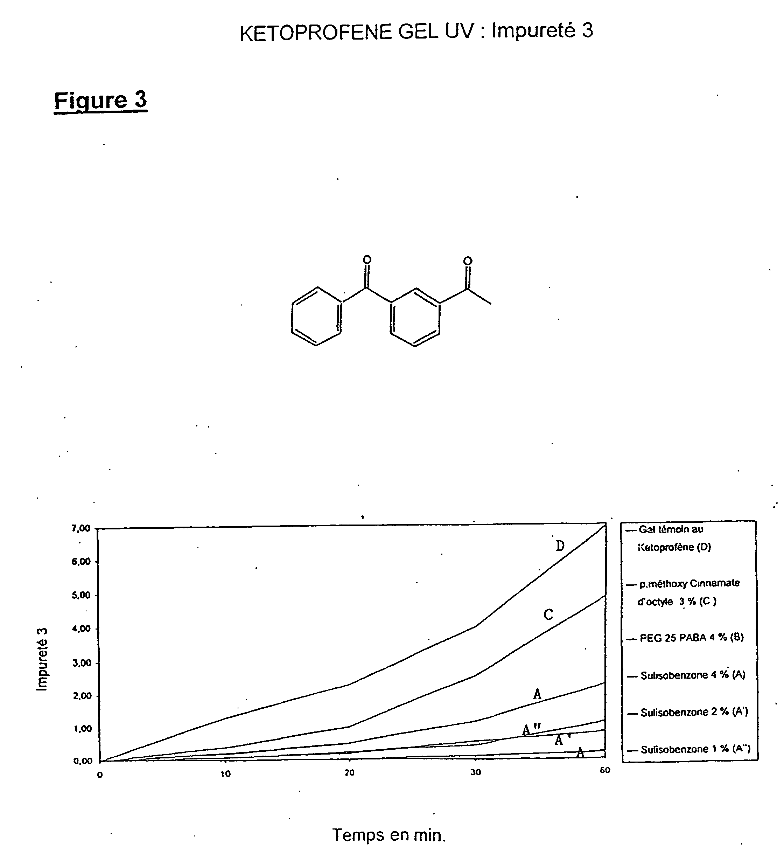

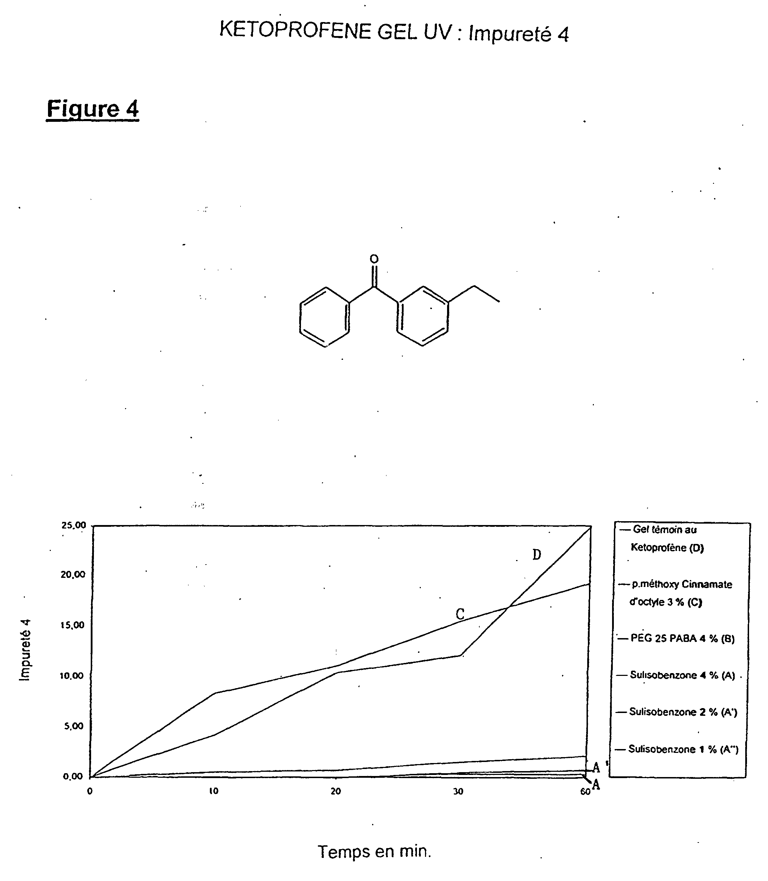

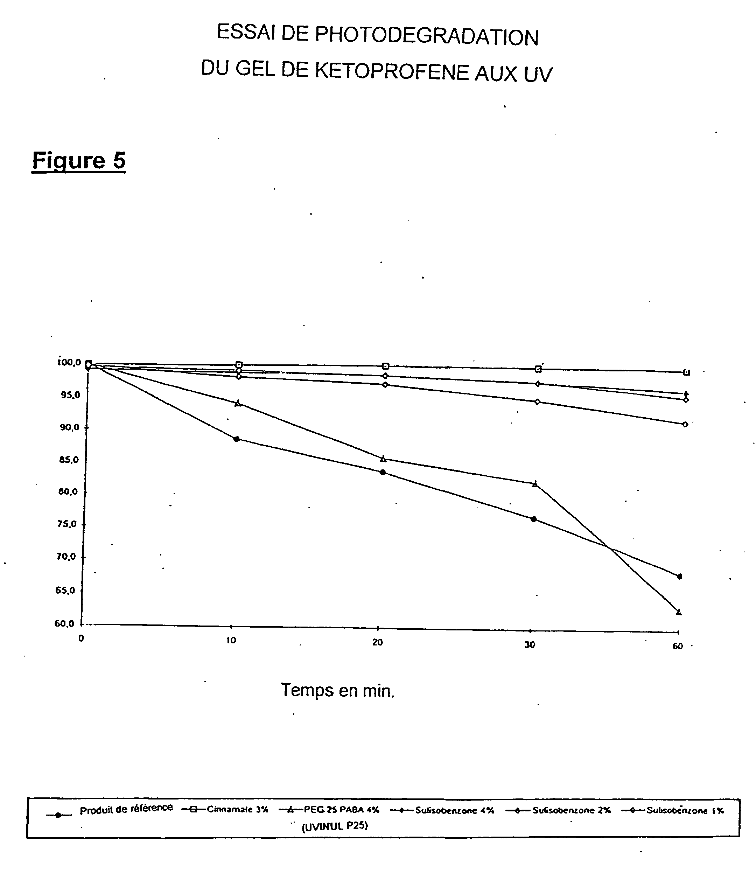

Les planches 1 à 5 mettent en évidence l'influence de l'agent photoprotecteur sur la dégradation de l'agent anti-inflammatoire.

Les compositions selon l'invention trouvent un emploi thérapeutique dans le traitement local des inflammations et/ou des douleurs articulaires.

## Revendications

1. Nouvelle composition pharmaceutique sous forme de gel aqueux renfermant, à titre de principe actif, le kétoprofène ou l'un de ses sels, associé à un agent filtrant les rayons U.V., choisi parmi le dérivé PEG 25 de l'acide p. aminobenzoïque et la sulisobenzone dans un excipient ou véhicule approprié.

2. Composition selon la revendication 1, dans laquelle le composé filtrant les rayons ultraviolets est un filtre pour au moins les rayons UV-B.

3. Composition selon la revendication 1 ou la revendication 2, dans laquelle la concentration en filtre est au moins égale à 0,5 %.

4. Composition pharmaceutique selon l'une des revendications 1 à 3, dans laquelle la concentration en filtre UV est comprise entre 0,5 et 10 %..

5. Composition pharmaceutique selon l'une des revendications 1 à 4, dans laquelle la concentration en filtre UV soluble est comprise entre 1 et 5 %..

6. Composition pharmaceutique selon l'une des revendications 1 à 5, dans laquelle la concentration en kétoprofène varie de 1 à 10 %.

7. Composition pharmaceutique selon la revendication 6, dans laquelle la concentration en kétoprofène varie entre 2 et 5 %.

8. Composition pharmaceutique selon la revendication 6 ou la revendication 7, dans laquelle le kétoprofène est utilisé sous forme acide ou sous forme de sel avec une base minérale ou organique

9. Composition pharmaceutique selon l'une des revendications 1 à 8, qui renferme en outre un parfum ou un agent odoriférant.

10. Composition pharmaceutique selon la revendication 9, dans laquelle le parfum ou l'agent odoriférant est choisi parmi l'essence de lavande, de géranium rosat, de lavandin, d'Ylang-Ylang et de citronnelle.

11. Composition pharmaceutique selon l'une des revendications 1 à 10, dans laquelle le véhicule ou l'excipient est un de ceux qui conviennent pour la réalisation d'un gel.

12. Composition pharmaceutique selon l'une des revendications 1 à 11, dans laquelle l'excipient est un agent gélifiant.

13. Composition pharmaceutique selon l'une des revendications 1 à 12, dans laquelle l'agent gélifiant est une dispersion de Carbomère ajustée à un pH s'échelonnant de 5 à 7 par addition d'un composé basique minéral ou organique.

14. Composition pharmaceutique selon l'une des revendications 1 à 13, qui renferme en outre un agent antioxydant et notamment le butylhydroxyanisole, le terbutylparacresol, l'ascorbate de palmityle ou les tocophérols.

15. Composition pharmaceutique selon l'une des revendications 1 à 14, qui renferme en outre un ou plusieurs alcools aliphatiques, et notamment l'éthanol.

## Claims

1. Novel pharmaceutical composition in the form of an aqueous gel containing as the active ingredient, Ketoprofen or one of its salts, in conjunction with a filtering agent for the UV rays selected among the PEG - 25 derivative of p.amino benzoic acid and sulisobenzone, in a suitable excipient or vehicle.

2. Composition according to claim 1, wherein the filtering agent for the UV rays is a filter for at least the UV-B rays.

3. Compositions according to claim 1 or claim 2, wherein the content of filter is at least of 0,5 %.

4. Pharmaceutical composition according to any of claims 1 to 3, wherein the content of UV filter lies between 0,5 and 10 %.

5. Pharmaceutical composition according to any of claims 1 to 4, wherein the content of soluble UV - filter lies between 1 and 5 %.

6. Pharmaceutical composition according to any of claims 1 to 5, wherein the content of Ketoprofen ranges from 1 to 10 %.

7. The pharmaceutical composition according to claim 6, wherein the content of Ketoprofen ranges between 2 and 5 %.

8. The pharmaceutical composition according to claim 6 or claim 7, wherein Ketoprofen is utilized in the acidic form or in the form of a salt with an anorganic or organic base.

9. Pharmaceutical composition according to anyone of claims 1 to 8, which further contains a perfume or an aromatising agent.

10. The pharmaceutical composition according to claim 9, wherein the perfume on the aromatising agent is selected among lavander oil, pelargonium oil, Lavendula Abrialli oil, Ylang-Ylang oil and Balm oil.

11. Pharmaceutical composition according to anyone of claims 1 to 10, wherein the vehicle or excipient is one of those suitable for the achievement of a gel.

12. Pharmaceutical composition according to anyone of claims 1 to 11, wherein the excipient is a gellifying agent.

13. Pharmaceutical composition according to anyone of claims 1 to 12, wherein the gellifying agent is a dispersion of Carbomer adjusted to a pH value ranging from 5 to 7, by adding an anorganic or organic basic component.

14. Pharmaceutical composition according to anyone of claims 1 to 13, which further contains an anti-oxydizing agent and namely butylhydroxyamisole, terbutylparacresol, palmityl ascorbate or the Tocopherols.

15. Pharmaceutical composition according to anyone of claims 1 to 14, which further contains one or more aliphatic alcohols and namely ethanol.

## Patentansprüche

1. Neue pharmazeutische Zusammensetzung in Form eines wässrigen Cels, enthaltend als Wlrkstoff Ketoprofen oder eines seiner Salze in Verbindung mit einem die UV-Strahlen filternden Stoff, gewählt unter dem Derivat PEG 25 der p-Aminobenzoesäure und Sulisobenzon, in einer geeigneten Grundmasse oder einem geeigneten Träger.

2. Zusammensetzung nach Anspruch 1, in der die die ultravioletten Strahlen filternde Verbindung ein Filter für mindestens die UV-B-Strahlen ist.

3. Zusammensetzung nach Anspruch 1 oder 2, in der die Konzentration des Filters mindestens gleich 0,5 % ist.

4. Pharmazeutische Zusammensetzung nach einem der Ansprüche 1 bis 3, in der die Konzentration des UV-Filters zwischen 0,5 und 10 % liegt.

5. Pharmazeutische Zusammensetzung nach einem der Ansprüche 1 bis 4, in der die Konzentration des löslichen UV-Filters zwischen 1 und 5 % liegt.

6. Pharmazeutische Zusammensetzung nach einem der Ansprüche 1 bis 5, in der die Konzentration von Ketoprofen von 1 bis 10 % schwankt.

7. Pharmazeutische Zusammensetzung nach Anspruch 6, in der die Konzentration von Ketoprofen zwischen 2 und 10 % schwankt.

8. Pharmazeutische Zusammensetzung nach Anspruch 6 oder Anspruch 7, in der Ketoprofen in saurer Form oder in Form eines Salzes mit einer mineralischen oder organischen Base verwendet wird.

9. Pharmazeutische Zusammensetzung nach einem der Ansprüche 1 bis 8, die außerdem ein Parfüm oder einen Duftstoff enthält.

10. Pharmazeutische Zusammensetzung nach Anspruch 9, in der das Parfüm oder dor Duftstoff untor Lavondol , Parfümgoranium , Lavandin , Ylang-Ylang- und Zitronenkrautessenz gewählt wird.

11. Pharmazeutische Zusammensetzung nach einem der Ansprüche 1 bis 10, in der der Träger oder die Grundmasse einer bzw. eine von jenen ist, die für die Herstellung eines Gels geeignet sind.

12. Pharmazeutische Zusammensetzung nach einem der Ansprüche 1 bis 11, in der die Grundmasse ein Geliermittel ist.

13. Pharmazeutische Zusammensetzung nach einem der Ansprüche 1 bis 12, in der das Geliermittel eine Carbomer-Dispersion ist, die durch Zusatz einer mineralischen oder organischen basischen Verbindung auf einen pH-Wert von 5 bis 7 eingestellt ist.

14. Pharmazeutische Zusammensetzung nach einem der Ansprüche 1 bis 13, die außerdem einen oxidationshemmenden Stoff enthält, insbesondere Butylhydroxyanisol, Terbutylparacresol, Palmitylascorbat oder Tocopherole.

15. Pharmazeutische Zusammensetzung nach einem der Ansprüche 1 bis 14, die außerdem einen oder mehrere aliphatische Alkohole, insbesondere Ethanol, enthält.
